# EUROPÄISCHE PATENTANMELDUNG

(11) **EP 0 718 305 A1**
(43) Veröffentlichungstag der Anmeldung: **26.06.1996**
(21) Anmeldenummer: 95119897.7
(22) Anmeldetag: 16.12.1995
(51) Int. Cl.: C07H 15/04

(54) **Verfahren zur Herstellung von Aldobionsäureamid**

(30) Priorität: 24.12.1994 DE 4446632
(71) Anmelder: Solvay Deutschland GmbH, D-30173 Hannover (DE)
(72) Erfinder: Gerling, Klaus-Günther, D-30880 Laatzen (DE); Schreer, Claudia, D-31174 Schellerten (DE); Schwarz, Petra, D-30449 Hannover (DE); Wendler, Kornelia, D-31319 Sehnde (DE)
(74) Vertreter: Lauer, Dieter, Dr.

(57) **Zusammenfassung**

Beschrieben wird ein Verfahren zur Herstellung von Aldobionsäureamiden direkt aus Aldobionsäure als Ausgangssubstanz, bei dem man eine Aldobionsäure durch Wasserentfernung mittels azeotroper Destillation zum Aldobionsäurelacton umsetzt, welches man anschließend ohne weitere Aufreinigungs- und Isolierungsschritte mit Aminen zum Aldobionsäureamid reagieren läßt.

## Beschreibung

Die Anmeldung betrifft ein Verfahren zur Herstellung von Aldobionsäureamid.

Aus der EP 569 869 ist ein Verfahren zur Herstellung von Lactobionsäureamidzusammensetzungen bekannt, bei dem man ausgehend vom Lactobionsäurelacton diese Ausgangsverbindung mit den entsprechenden Fettaminen in einem niederen Alkylalkohol als Lösungsmittel reagieren läßt. In der DE 11 55 771 wird ein Verfahren zur Herstellung von Aldobionsäurealkylamiden, insbesondere von Maltobionsäure-, Lactobionsäure- oder Cellobionsäurealkylamiden vorgeschlagen, bei dem man die entsprechenden Säurelactone mit Alkylaminen in Dimethylformamid als Lösungsmittel umsetzt.

Nach den bisher bekannten Verfahren des Standes der Technik zur Herstellung von Aldobionsäureamid wird als Ausgangskomponente immer das Aldobionsäurelacton eingesetzt. Aldobionsäurelacton muß dabei zuvor in einer vorgeschalteten Verfahrensstufe erst durch Wasserabspaltung der entsprechenden Aldobionsäure hergestellt und isoliert werden. Aufgrund der anwendungstechnisch interessanten Eigenschaften der Aldobionsäureamide wäre es wünschenswert, wenn Aldobionsäureamide direkt ausgehend von den Aldobionsäuren durch ein einstufiges Verfahren hergestellt werden könnten.

Der vorliegenden Erfindung liegt daher die Aufgabe zugrunde, ein Verfahren zur Verfügung zu stellen, nach welchem sich Aldobionsäureamide direkt aus Aldobionsäuren herstellen lassen.

Gegenstand der Erfindung ist somit ein Verfahren zur Herstellung von Aldobionsäureamid, bei dem man
a) eine Aldobionsäure oder eine Aldobionsäurelösung mit einem zur Azeotropbildung mit Wasser befähigten organischen Lösungsmittel zusammengibt,
b) die Aldobionsäure durch Wasserentfernung mittels azeotroper Destillation zum Aldobionsäurelacton umsetzt und
c) das Aldobionsäurelacton mit Aminen zum Aldobionsäureamid reagieren läßt.

Mit dem erfindungsgemäßen Verfahren wird eine Direktsynthese von Aldobionsäureamiden aus Aldobionsäuren als Ausgangskomponenten ermöglicht, ohne daß dabei das Zwischenprodukt Aldobionsäurelacton isoliert und aufgearbeitet werden muß. Da somit Aufarbeitungsschritte zur Isolierung von Aldobionsäurelacton entfallen, ermöglicht das erfindungsgemäße Verfahren eine besonders einfache und wirtschaftliche Reaktionsdurchführung. Das Verfahren kann dabei sowohl nichtkontinuierlich als auch vorzugsweise kontinuierlich durchgeführt werden.

Im erfindungsgemäßen Verfahren können die Aldobionsäuren entweder direkt als Festsubstanz oder als Lösung in Wasser oder einem organischen Lösungsmittel eingesetzt werden. Bevorzugt setzt man wäßrige Aldobionsäurelösungen ein. Dies ist besonders vorteilhaft, da somit wäßrige Aldobionsäurelösungen, wie sie aus der Synthese der Aldobionsäuren aus den jeweiligen Aldosen anfallen, direkt für die Weiterreaktion zu den entsprechenden Säureamiden verwendet werden können.

Nach dem erfindungsgemäßen Verfahren lassen sich alle bekannten Aldobionsäuren wie z. B. Maltobionsäure, Lactobionsäure oder Cellobionsäure umsetzen. Vorzugsweise läßt man Lactobionsäure nach dem erfindungsgemäßen Verfahren zu den jeweiligen Lactobionsäureamiden reagieren.

Im Verfahrensschritt a) des erfindungsgemäßen Verfahrens mischt man Aldobionsäure oder eine Aldobionsäurelösung mit einem organischen Lösungsmittel, welches ein Azeotrop mit Wasser bildet. Beispielsweise können zur Azeotropbildung befähigte Alkylalkohole, Essigsäureester oder auch Cyclohexan als organisches Lösungsmittel eingesetzt werden. Bevorzugt verwendet man organische Lösungsmittel aus der Gruppe Ethylacetat, Cyclohexan, 2-Methoxy-Ethanol und 2-Ethoxy-Ethanol. Besonders bevorzugt setzt man 2-Ethoxy-Ethanol ein.

Zweckmäßigerweise führt man das erfindungsgemäße Verfahren so durch, daß man in Verfahrensschritt a) die Aldobionsäure bzw. die Aldobionsäurelösung mit der gleichen bzw. doppelt bis fünffachen Volumenmenge eines organischen Lösungsmittels, welches mit Wasser ein Azeotrop bilden kann, mischt und für ca. 1 Stunde bei erhöhter Temperatur, z. B. 70 bis 80 °C rühren läßt. Anschließend wird in Verfahrensschritt b) eine azeotrope Destillation gegebenenfalls unter Vakuum durchgeführt, wobei als Destillat ein azeotropes Gemisch aus Wasser und dem organischen Lösungsmittel abgetrennt wird. Zur vollständigen Umsetzung der Aldobionsäure zum Aldobionsäurelacton kann gewünschtenfalls, nachdem bis zu ca. 75 % der vorgelegten Volumenmenge abdestilliert worden ist, frisches organisches Lösungsmittel erneut in der 0,3 bis 2-fachen Menge, bezogen auf das ursprünglich eingesetzte Gemisch aus organischem Lösungsmittel und Aldobionsäure bzw. Aldobionsäurelösung, hinzugegeben werden und die azeotrope Destillation weitergeführt werden. Die Zugabe von frischem organischen Lösungsmittel kann gewünschtenfalls mehrfach wiederholt werden. Die azeotrope Destillation wird möglichst so lange durchgeführt, bis aus dem Gemisch Wasser möglichst vollständig herausgeschleppt ist. Anschließend wird in Verfahrensschritt c) das gewünschte Amin, direkt, falls dieses flüssig sein sollte, oder gelöst im gleichen organischen Lösungsmittel, zu der erhaltenen Suspension hinzugegeben und für mindestens 30 Minuten oder auch länger bei erhöhter Temperatur, z. B. 40 bis 50 °C gerührt, wobei sich üblicherweise aus der Suspension wieder eine nahezu klare Lösung bildet. Anschließend wird noch für mindestens ca. 12 Stunden bei Raumtemperatur gerührt. Dann wird ein weiteres organisches Lösungsmittel, z. B. Ethylacetat, hinzugegeben. Ein Niederschlag fällt aus, der abfiltriert, gewaschen und dann im Vakuumtrockenschrank bei Raumtemperatur oder vorzugsweise 40 bis 50 °C getrocknet wird. Die Ausbeuten an Aldobionsäurealkylamid liegen üblicherweise im Bereich von 85 bis 98 Gew.-%.

Nach dem erfindungsgemäßen Verfahren lassen sich vorzugsweise Aldobionsäurealkylamide mit Alkylketten jeder gewünschten Kettenlänge herstellen. Bevorzugt setzt man hierfür Alkylamine mit einer Alkylkettenlänge von 8 bis 18 Kohlenstoffatomen ein. Besonders bevorzugt verwendet man primäre Fettamine, welche aus natürlich vorkommenden Fettsäuren gewonnen wurden. Üblicherweise liegen derartige primäre Fettamine als Gemische von Fettaminen unterschiedlicher Kettenlänge vor. Derartige Fettamingemische enthalten neben gesättigten Fettaminen zumeist noch einen Anteil von einfach bzw. mehrfach ungesättigten Fettaminen. Der eine Teil von einfach ungesättigten Fettaminen kann dabei in diesen Fettamingemischen zwischen ca. 5 und 85 Gew.-% schwanken. Beispiele solcher Fettamingemische sind Kokosfettamin aus dem aus Kokosfett stammenden Fettsäuregemisch, Talgamin und hydriertes Talgamin aus dem aus Talg stammenden Fettsäuregemisch, Oleylamin aus dem aus Sonnenblumenöl und/oder Sojaöl stammenden Fettsäuregemisch. Insbesondere setzt man im erfindungsgemäßen Verfahren Fettamine aus der Gruppe Kokosfettamin, Talgamin, hydriertes Talgamin und Oleylamin ein.

In einer bevorzugten Variante wird das erfindungsgemäße Verfahren so durchgeführt, daß man das in Verfahrensschritt b) erhaltene abdestillierte azeotrope Gemisch und das in Verfahrensschritt c) erhaltene Lösungsmittelgemisch miteinander vereinigt und wieder in Wasser und organische Lösungsmittelkomponenten auftrennt und die organischen Lösungsmittel wieder in Verfahrensschritt a) oder in Verfahrensschritt c) zurückführt. Die Auftrennung kann dabei auf an sich bekannte Weise erfolgen. Bei Verwendung von z. B. Ethylacetat als organischem Lösungsmittel kann ein Wasser-Ethylacetat-Gemisch abdestilliert und aus dem Destillat auf an sich bekannte Weise durch Phasentrennung die Wasserphase mittels eines bekannten Wasserabscheiders oder Scheidetrichters vom Ethylacetat abgetrennt werden. Auch alle anderen Methoden zur Wasserabtrennung sind möglich, wie z. B. die Auftrennung über eine Kolonnen-Destillation oder durch Extraktion oder Perforation.

Durch die Wiederrückführung der zurückgewonnenen organischen Lösungsmittel kann das erfindungsgemäße Verfahren in dieser bevorzugten kontinuierlichen Prozeßführung in ökonomischer und umweltfreundlicher Weise als weitgehend geschlossener Kreislauf-Prozeß ausgestaltet werden. Da somit die organischen Lösungsmittel immer wieder in den Prozeß zurückgeführt werden, wird in vorteilhafter Weise der Anfall größerer zu entsorgender Lösungsmittelrückstände vermieden. Da das erfindungsgemäße Verfahren als Ein-Stufen-Verfahren ohne Aufarbeitung eines Zwischenproduktes durchgeführt wird, ermöglicht es in überraschender Weise einen sehr einfachen Zugang zu den Aldobionsäureamiden direkt aus den Aldobionsäuren als Ausgangsverbindungen.

Die nachfolgenden Beispiele sollen die Erfindung weiter erläutern, ohne sie jedoch in ihrem Umfang zu begrenzen.

### Beispiele

Exemplarisch wird das erfindungsgemäße Verfahren zur Herstellung von Aldobionsäureamid an der Herstellung von Lactobionsäure-N-oleylamid beschrieben.

### 1. Herstellung von Lactobionsäure-N-Oleylamid:

Es wurden 50 g einer wäßrigen Lösung mit einem Gehalt von 77 Gew.-% Lactobionsäure (entsprechend 0,108 Mol) mit 200 ml 2-Ethoxy-Ethanol in einem Destillationskolben vermischt und für ca. 1 Stunde bei 70 bis 80 °C gerührt. Anschließend wurde unter Vakuum ein azeotropes Gemisch aus 2-Ethoxy-Ethanol und Wasser in einer Volumenmenge von ca. 150 ml abdestilliert. Nach Zugabe von weiteren 100 ml 2-Ethoxy-Ethanol wurde die azeotrope Destillation bis auf eine Suspension von ca. 60 ml fortgeführt. Man ließ die Suspension auf c. 50 °C abkühlen. 25,9 g Oleylamin (entsprechend 0,098 Mol) wurden dann in 20 ml 2-Ethoxy-Ethanol gelöst und langsam unter Rühren der Suspension hinzugefügt. Dann wurde für ca. 2 Stunden bei 50 °C gerührt, wobei sich wieder eine nahezu klare Lösung bildete. Für ca. 12 Stunden wurde dann noch bei Raumtemperatur gerührt. Hierbei bildete sich wieder erneut eine Suspension, welche in 500 ml Ethylacetat eingerührt wurde. Der dabei ausgefallene weiße Niederschlag wurde unter leichtem Vakuum abfiltriert, mit Ethylacetat gewaschen und dann im Vakuumtrockenschrank bei 40 bis 50 °C getrocknet. Die Ausbeute betrug 85 Gew.-% Lactobionsäure-N-Oleylamid, bezogen auf Oleylamin.
Die Aufarbeitung des zurückbleibenden Lösungsmittelgemisches erfolgte nach Vereinigung mit dem abdestillierten Azeotrop aus 2-Ethoxy-Ethanol und Wasser.
Hierfür wurde die Mischung aus Ethylacetat, 2-Ethoxy-Ethanol und Wasser bei 100 °C über eine Kolonne mit Wasserabscheider destilliert. In der Destillatvorlage erhielt man ein 2-Phasen-Gemisch aus Ethylacetat und Wasser, aus dem die wäßrige Phase über den Wasserabscheider entfernt wurde, während die organische Phase aus Ethylacetat wieder in das Verfahren zur Herstellung von Lactobionsäure-N-oleylamid zurückgeführt wurde. Nach vollständiger Entfernung von Wasser und Ethylacetat wurde das im Sumpf verbliebene 2-Ethoxy-Ethanol bei ca 80 °C im Vakuum destilliert und ebenfalls wieder in den Herstellprozeß zurückgeführt.

## Patentansprüche

1. Verfahren zur Herstellung von Aldobionsäureamid, dadurch gekennzeichnet, daß man
a) eine Aldobionsäure oder eine Aldobionsäurelösung mit einem zur Azeotropbildung mit Wasser befähigten organischen Lösungsmittel zusammengibt,
b) die Aldobionsäure durch Wasserentfernung mittels azeotroper Destillation zum Aldobionsäurelacton umsetzt und
c) das Aldobionsäurelacton mit Aminen zum Aldobionsäurealkylamid reagieren läßt.

2. Verfahren nach Anspruch 1, dadurch gekennzeichnet, daß man eine wäßrige Aldobionsäurelösung einsetzt.

3. Verfahren nach einem der vorherigen Ansprüche, dadurch gekennzeichnet, daß man als organisches Lösungsmittel Lösungsmittel aus der Gruppe Cyclohexan, Ethylacetat, 2-Methoxy-Ethanol, 2-Ethoxy-Ethanol, vorzugsweise 2-Ethoxy-Ethanol einsetzt.

4. Verfahren nach einem der vorherigen Ansprüche, dadurch gekennzeichnet, daß man als Amine Alkylamine einsetzt.

5. Verfahren nach Anspruch 4, dadurch gekennzeichnet,daß man primäre Alkylamine mit einer Kettenlänge im Bereich von 8 bis 18 Kohlenstoffatomen einsetzt.

6. Verfahren nach Anspruch 5, dadurch gekennzeichnet, daß man als primäre Amine Fettamine einsetzt.

7. Verfahren nach Anspruch 6, dadurch gekennzeichnet, daß man Fettamine aus der Gruppe Kokosfettamin, Talgamin, hydriertes Talgamin, Oleylamin einsetzt.

8. Verfahren nach einem der vorherigen Ansprüche, dadurch gekennzeichnet, daß man Aldobionsäurealkylamid, vorzugsweise Lactobionsäurealkylamid herstellt.

9. Verfahren nach Anspruch 1, dadurch gekennzeichnet, daß man das in Verfahrensschritt b) erhaltene azeotrope Destillat und das Lösungsmittelgemisch aus Verfahrensschritt c) in Wasser und organische Lösungsmittel auftrennt und die organischen Lösungsmittel wieder in die Verfahrensschritte a) und/oder c) zurückführt.

10. Verfahren nach Anspruch 1, dadurch gekennzeichnet, daß man das Verfahren kontinuierlich durchführt.
